# EUROPÄISCHE PATENTANMELDUNG

(11) **EP 1 118 666 A2**
(43) Veröffentlichungstag der Anmeldung: **25.07.2001**
(21) Anmeldenummer: 01100695.4
(22) Anmeldetag: 12.01.2001
(51) Int. Cl.: C12N 15/54, C12N 15/10, C12N 9/12, C12N 1/21, C12P 13/04, C12P 13/08

(54) **Für das ptsH-Gen codierende Nukleotidsequenzen aus Corynebacterium glutamicum**

(30) Priorität: 13.01.2000 DE 10001101
(71) Anmelder: Degussa-Hüls Aktiengesellschaft, 60287 Frankfurt am Main (DE)
(72) Erfinder: Farwick, Mike, Dr., 33615 Bielefeld (DE); Möckel, Bettina, Dr., 40597 Düsseldorf (DE); Pfefferle, Walter, Dr., 33790 Halle (DE)

(57) **Zusammenfassung**

Die Erfindung betrifft ein isoliertes Polynukleotid, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70 % identisch ist mit der Aminosäuresequenz von SEQ ID No.2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c),
und Verfahren zur fermentativen Herstellung von L-Aminosäuren unter Verstärkung des für die Komponente H des Phosphotransferase-Systems codierenden ptsH-Gens sowie die Verwendung der obigen Polynukleotide als Primer oder Hybridisierungssonde.

## Beschreibung

Gegenstand der Erfindung sind für ptsH codierende Nukleotidsequenzen und Verfahren zur fermentativen Herstellung von L-Aminosäuren, insbesondere L-Lysin, unter Verwendung von coryneformen Bakterien, in denen das ptsH-Gen verstärkt wird.

### Stand der Technik

L-Aminosäuren, insbesondere L-Lysin, finden in der Humanmedizin und in der pharmazeutischen Industrie, insbesondere aber in der Tierernährung Anwendung.

Es ist bekannt, daß L-Aminosäuren durch Fermentation von Stämmen coryneformer Bakterien, insbesondere Corynebacterium glutamicum hergestellt werden. Wegen der großen Bedeutung wird ständig an der Verbesserung der Herstellungsverfahren gearbeitet. Verfahrensverbesserungen können fermentationstechnische Maßnahmen wie z. B. Rührung und Versorgung mit Sauerstoff, oder die Zusammensetzung der Nährmedien wie z.B. die Zuckerkonzentration während der Fermentation, oder die Aufarbeitung zur Produktform durch z.B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Lysin-Analogon S-(2-Aminoethyl)-Cystein oder auxotroph für regulatorisch bedeutsame Metabolite sind und L-Lysin produzieren.

Seit einigen Jahren werden ebenfalls Methoden der rekombinanten DNA-Technik zur Stammverbesserung L-Aminosäuren produzierender Stämme von Corynebacterium eingesetzt, indem man einzelne Biosynthesegene für L-Aminosäuren amplifiziert und die Auswirkung auf die L-Aminosäure-Produktion untersucht. Übersichtsartikel hierzu findet man unter anderem bei Kinoshita ("Glutamic Acid Bacteria", in: Biology of Industrial Microorganisms, Demain and Solomon (Eds.), Benjamin Cummings, London, UK, 1985, 115-142), Hilliger (BioTec 2, 40-44 (1991)), Eggeling (Amino Acids 6:261-272 (1994)), Jetten und Sinskey (Critical Reviews in Biotechnology 15, 73-103 (1995)) und Sahm et al. (Annuals of the New York Academy of Science 782, 25-39 (1996)).

### Aufgabe der Erfindung

Die Erfinder haben sich zur Aufgabe gestellt, neue Maßnahmen zur verbesserten fermentativen Herstellung von L-Aminosäuren, insbesondere L-Lysin, bereitzustellen.

### Beschreibung der Erfindung

L-Aminosäuren, insbesondere L-Lysin, finden in der Humanmedizin, in der pharmazeutischen Industrie und insbesondere in der Tierernährung Anwendung. Es besteht daher ein allgemeines Interesse daran, neue verbesserte Verfahren zur Herstellung von L-Aminosäuren, insbesondere L-Lysin, bereitzustellen.

Wenn im folgenden L-Lysin oder Lysin erwähnt werden, sind damit nicht nur die Base sondern auch die Salze wie z. B. Lysin-Monohydrochlorid oder Lysin-Sulfat gemeint.

Gegenstand der Erfindung ist ein isoliertes Polynukleotid aus coryneformen Bakterien, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70% identisch ist mit der Aminosäuresequenz von SEQ ID No.2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c).

Gegenstand der Erfindung ist ebenfalls ein Polynukleotid, das eine in coryneformen Bakterien replizierbare, bevorzugt rekombinante, DNA ist.

Gegenstand der Erfindung ist ebenfalls ein Polynukleotid, das eine RNA ist.

Gegenstand der Erfindung ist ebenfalls ein Polynukleotid, wobei es sich bevorzugt um eine replizierbare DNA handelt, enthaltend:
(i) die Nukleotidsequenz, gezeigt in SEQ ID No.1, oder
(ii) mindestens eine Sequenz, die der Sequenz (i) innerhalb des Bereichs der Degeneration des genetischen Codes entspricht, oder
(iii) mindestens eine Sequenz, die mit der zur Sequenz (i) oder (ii) komplementären Sequenz hybridisiert, und gegebenenfalls
(iv) funktionsneutrale Sinnmutationen in (i).

Weitere Gegenstände sind
ein Vektor, enthaltend eines der genannten Polynukleotide,
und als Wirtszelle dienende coryneforme Bakterien, die den Vektor enthalten.

Gegenstand der Erfindung sind ebenso Polynukleotide, die im wesentlichen aus einer Polynukleotidsequenz bestehen, die erhältlich sind durch Screening mittels Hybridisierung einer entsprechenden Genbank, die das vollständige Gen mit der Polynukleotidsequenz entsprechend SEQ ID No. 1 enthält, mit einer Sonde, die die Sequenz des genannten Polynukleotids gemäß SEQ ID No. 1 oder ein Fragment davon enthält und Isolierung der genannten DNA-Sequenz.

Polynukleotidsequenzen gemäß der Erfindung sind geeignet als Hybridisierungs-Sonden für RNA, cDNA und DNA, um cDNA in voller Länge zu isolieren, die für die Komponente H des Phosphotransferase-Systems (ptsH) codieren und solche cDNA oder Gene zu isolieren, die eine hohe Ähnlichkeit der Sequenz mit der des Gens für die Komponente H des Phosphotransferase-Systems aufweisen.

Polynukleotidsequenzen gemäß der Erfindung sind weiterhin geeignet als Primer zur Herstellung von DNA von Genen, die für die Komponente H des Phosphotransferase-Systems codieren, durch die Polymerase-Kettenreaktion (PCR).

Solche als Sonden oder Primer dienende Oligonukleotide enthalten mindestens 30, bevorzugt mindestens 20, ganz besonders bevorzugt mindestens 15 aufeinanderfolgende Nukleotide. Geeignet sind ebenfalls Oligonukleotide mit einer Länge von mindestens 40 oder 50 Nukleotiden.

"Isoliert" bedeutet aus seinem natürlichen Umfeld herausgetrennt.

"Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Unter "Polypeptiden" versteht man Peptide oder Proteine, die zwei oder mehr über Peptidbindungen verbundene Aminosäuren enthalten.

Die Polypeptide gemäß Erfindung schließen ein Polypeptid gemäß SEQ ID No. 2 ein, weiterhin solche mit der biologischen Aktivität der Komponente H des Phosphotransferase-Systems und auch solche, die zu wenigstens 70 % identisch sind mit dem Polypeptid gemäß SEQ ID No. 2, bevorzugt zu wenigstens 80% und besonders die zu wenigstens 90 % bis 95 % Identität mit dem Polypeptid gemäß SEQ ID No. 2 und die genannte Aktivität aufweisen.

Die Erfindung betrifft weiterhin ein Verfahren zur fermentativen Herstellung von L-Aminosäuren, insbesondere L-Lysin, unter Verwendung von coryneformen Bakterien, die insbesondere bereits eine L-Aminosäure produzieren, und in denen die für das ptsH-Gen codierenden Nukleotidsequenzen verstärkt, insbesondere überexprimiert werden.

Der Begriff "Verstärkung" beschreibt in diesem Zusammenhang die Erhöhung der intrazellulären Aktivität eines oder mehrerer Enzyme in einem Mikroorganismus, die durch die entsprechende DNA codiert werden, indem man beispielsweise die Kopienzahl des Gens bzw. der Gene erhöht, einen starken Promotor verwendet oder ein Gen verwendet, das für ein entsprechendes Enzym mit einer hohen Aktivität codiert und gegebenenfalls diese Maßnahmen kombiniert.

Die Mikroorganismen, die Gegenstand der vorliegenden Erfindung sind, können L-Aminosäuren, insbesondere L-Lysin aus Glucose, Saccharose, Lactose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind zum Beispiel die bekannten Wildtypstämme
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 und
Brevibacterium divaricatum ATCC14020
und daraus hergestellte L-Lysin produzierende Mutanten bzw. Stämme, wie beispielsweise
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 und
Corynebacterium glutamicum DSM5715.

Den Erfindern gelang es, das neue, für die Komponente H des Phosphotransferase-Systems codierende ptsH-Gen von C. glutamicum zu isolieren.

Zur Isolierung des ptsH-Gens oder auch anderer Gene von C. glutamicum wird zunächst eine Genbank dieses Mikroorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495-508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E. coli K-12 Stamm NM554 (Raleigh et al., 1988, Nucleic Acids Research 16:1563-1575) angelegt wurde. Börmann et al. (Molecular Microbiology 6(3), 317-326 (1992)) wiederum beschreiben eine Genbank von C. glutamicum ATCC13032 unter Verwendung des Cosmids pHC79 (Hohn und Collins, Gene 11, 291-298 (1980)). Zur Herstellung einer Genbank von C. glutamicum in E. coli können auch Plasmide wie pBR322 (Bolivar, Life Sciences, 25, 807-818 (1979)) oder pUC9 (Vieira et al., 1982, Gene, 19:259-268) verwendet werden. Als Wirte eignen sich besonders solche E. coli-Stämme, die restriktions- und rekombinationsdefekt sind. Ein Beispiel hierfür ist der Stamm DH5αMCR, der von Grant et al. (Proceedings of the National Academy of Sciences USA, 87 (1990) 4645-4649) beschrieben wurde. Die mit Hilfe von Cosmiden klonierten langen DNA-Fragmente können anschließend wiederum in gängige, für die Sequenzierung geeignete Vektoren subkloniert und anschließend sequenziert werden, so wie es z. B. bei Sanger et al. (Proceedings of the National Academy of Sciences of the United States of America, 74:5463-5467, 1977) beschrieben ist.

Auf diese Weise wurde die neue für ptsH codierende DNA-Sequenz von C. glutamicum erhalten, die als SEQ ID No. 1 Bestandteil der vorliegenden Erfindung ist. Weiterhin wurde aus der vorliegenden DNA-Sequenz mit den oben beschriebenen Methoden die Aminosäuresequenz des entsprechenden Proteins abgeleitet. In SEQ ID No. 2 ist die sich ergebende Aminosäuresequenz des ptsH-Genproduktes dargestellt.

Codierende DNA-Sequenzen, die sich aus SEQ ID No. 1 durch die Degeneriertheit des genetischen Codes ergeben, sind ebenfalls Bestandteil der Erfindung. In der Fachwelt sind weiterhin konservative Aminosäureaustausche wie z. B. Austausch von Glycin gegen Alanin oder von Asparaginsäure gegen Glutaminsäure in Proteinen als "Sinnmutationen" (sense mutations) bekannt, die zu keiner grundsätzlichen Veränderung der Aktivität des Proteins führen, d. h. funktionsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie. Aminosäuresequenzen, die sich in entsprechender Weise aus SEQ ID NO. 2 ergeben, und diese Aminosäuresequenzen codierende DNA-Sequenzen sind ebenfalls Bestandteil der Erfindung.

In gleicher Weise sind DNA-Sequenzen, die mit SEQ ID No. 1 oder Teilen von SEQ ID No. 1 hybridisieren Bestandteil der Erfindung. Schließlich sind DNA-Sequenzen Bestandteil der Erfindung, die durch die Polymerase-Kettenreaktion (PCR) unter Verwendung von Primern hergestellt werden, die sich aus SEQ ID No. 1 ergeben. Derartige Oligonukleotide haben typischerweise eine Länge von mindestens 15 Nukleotiden.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260). Anleitungen zur Amplifikation von DNA-Sequenzen mit Hilfe der Polymerase-Kettenreaktion (PCR) findet der Fachmann unter anderem im Handbuch von Gait: Oligonukleotide synthesis: a practical approach (IRL Press, Oxford, UK, 1984) und bei Newton und Graham: PCR (Spektrum Akademischer Verlag, Heidelberg, Deutschland, 1994).

Die Erfinder fanden heraus, daß coryneforme Bakterien nach Überexpression des ptsH-Gens in verbesserter Weise L-Aminosäuren insbesondere L-Lysin produzieren.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Aminosäure-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

Beispielhaft wurde das erfindungsgemäße ptsH-Gen mit Hilfe von Plasmiden überexprimiert.

Als Plasmide eignen sich solche, die in coryneformen Bakterien repliziert werden. Zahlreiche bekannte Plasmidvektoren wie z. B. pZ1 (Menkel et al., Applied and Environmental Microbiology (1989) 64: 549-554), pEKExl (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie z. B. solche, die auf pCG4 (US-A 4,489,160) oder pNG2 (Serwold-Davis et al., FEMS Microbiology Letters 66, 119-124 (1990)) oder pAG1 (US-A 5,158,891) beruhen, können in gleicher Weise verwendet werden.

Weiterhin eignen sich solche Plasmidvektoren mit Hilfe derer man das Verfahren der Genamplifikation durch Integration in das Chromosom anwenden kann, so wie es beispielsweise von Reinscheid et al. (Applied and Environmental Microbiology 60, 126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen bespielsweise pSUP301 (Simon et al., Bio/Technology 1, 784-791 (1983)), pK18mob oder pK19mob (Schäfer et al., Gene 145, 69-73 (1994)), pGEM-T (Promega corporation, Madison, WI, USA), pCR2.1-TOPO (Shuman (1994). Journal of Biological Chemistry 269:32678-84; US-Patent 5,487,993), pCR®Blunt (Firma Invitrogen, Groningen, Niederlande; Bernard et al., Journal of Molecular Biology, 234: 534-541 (1993)) oder pEM1 (Schrumpf et al, 1991, Journal of Bacteriology 173:4510-4516) in Frage. Der Plasmidvektor, der das zu amplifizierende Gen enthält, wird anschließend durch Konjugation oder Transformation in den gewünschten Stamm von C. glutamicum überführt. Die Methode der Konjugation ist beispielsweise bei Schäfer et al. (Applied and Environmental Microbiology 60, 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiological Letters 123, 343-347 (1994)) beschrieben. Nach homologer Rekombination mittels eines "cross over"-Ereignisses enthält der resultierende Stamm mindestens zwei Kopien des betreffenden Gens.

Ein weiterer Gegenstand der Erfindung ist demnach ein Verfahren zur fermentativen Herstellung von L-Aminosäuren, insbesondere L-Lysin, bei dem man einen mit einem Plasmidvektor transformierten Stamm einsetzt, und der Plasmidvektor die Nukleotidsequenz des für die Komponente H des Phosphotransferase-Systems codierenden Gens trägt.

Zusätzlich kann es für die Produktion von L-Aminosäuren, insbesondere L-Lysin, vorteilhaft sein, neben dem ptsH-Gen weitere Gene des Biosyntheseweges der gewünschten L-Aminosäure zu verstärken, damit eines oder mehrere Enzyme des jeweiligen Biosyntheseweges, der Glykolyse, der Anaplerotik oder des Aminosäure-Exports überexprimiert wird.

So kann beispielsweise für die Herstellung von L-Lysin gleichzeitig eines oder mehrere der Gene, ausgewählt aus der Gruppe
- das für die Dihydrodipicolinat-Synthase codierende dapA-Gen (EP-B 0 197 335),
- das für die Glyceraldehyd-3-Phosphat-Dehydrogenase codierende gap-Gen (Eikmanns (1992), Journal of Bacteriology 174:6076-6086),
- das für die Triosephosphat-Isomerase codierende tpi-Gen(Eikmanns (1992), Journal of Bacteriology 174:6076-6086),
- das für die 3-Phosphoglycerat-Kinase codierende pgk-Gen(Eikmanns (1992), Journal of Bacteriology 174:6076-6086),
- das für die Komponente M des Phosphoenolpyruvat-Zucker-Phosphotransferase Systems (ptsM) codierende ptsM-Gen (Lee et al. (1994), FEMS Microbiology Letters 1-2, 137-145),
- das für die Pyruvat-Carboxylase codierende pyc-Gen (DE-A-198 31 609), und
- das für den Lysin-Export codierende lysE-Gen (DE-A-195 48 222),
überexprimiert werden.

Weiterhin kann es für die Produktion von L-Aminosäuren, insbesondere L-Lysin, vorteilhaft sein, neben dem ptsH-Gen gleichzeitig
- das für die Phosphoenolpyruvat-Carboxykinase codierende pck-Gen (DE 199 50 409.1, DSM 13047) und/oder
- das für die Glucose-6-Phosphat-Isomerase codierende pgi-Gen (US 09/396,478, DSM 12969)
- das für die Pyruvat-Oxidase codierende poxB Gen (DE 19846499.1; DSM 13114)
abzuschwächen.

Weiterhin kann es für die Produktion von L-Aminosäuren, insbesondere L-Lysin, vorteilhaft sein, neben der Überexpression des ptsH-Gens unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die erfindungsgemäß hergestellten Mikroorganismen können kontinuierlich oder diskontinuierlich im Batch-Verfahren (Satzkultivierung) oder im Fed batch- (Zulaufverfahren) oder Repeated fed batch-Verfahren (repetitives Zulaufverfahren) zum Zwecke der Produktion von L-Aminosäuren, insbesondere L-Lysin, kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag,
Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z. B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische stickstoffhaltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden natriumhaltigen Salze verwendet werden. Das Kulturmedium muß weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten werden Sauerstoff oder sauerstoffhaltige Gasmischungen wie z. B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum an L-Lysin gebildet hat. Dieses Ziel wird normalerweise innerhalb von 10 Stunden bis 160 Stunden erreicht.

Ein weiterer Gegenstand der Erfindung ist demnach ein Verfahren zur fermentativen Herstellung von L-Aminosäuren, insbesondere L-Lysin, bei dem man folgende Schritte durchführt:
a) Fermentation von die L-Aminosäure produzierenden coryneformen Bakterien, in denen man zumindest das für die Komponente H des Phosphotransferase-Systems codierende ptsH-Gen verstärkt, insbesondere überexprimiert.
b) Anreicherung der L-Aminosäure im Medium oder in den Zellen der Bakterien und
c) Isolieren der L-Aminosäure.

Die Analyse von L-Lysin kann durch Anionenaustauschchromatographie mit anschließender Ninhydrin Derivatisierung erfolgen, so wie bei Spackman et al. (Analytical Chemistry, 30, (1958), 1190) beschrieben.

Das erfindungsgemäße Verfahren dient zur fermentativen Herstellung von L-Aminosäuren, insbesondere L-Lysin.

### Beispiele

Die vorliegende Erfindung wird im folgenden anhand von Ausführungsbeispielen näher erläutert.

### Beispiel 1

### Herstellung einer genomischen Cosmid-Genbank aus Corynebacterium glutamicum ATCC 13032

Chromosomale DNA aus Corynebacterium glutamicum ATCC 13032 wurde wie bei Tauch et al. (1995, Plasmid 33:168-179) beschrieben isoliert und mit dem Restriktionsenzym Sau3AI (Amersham Pharmacia, Freiburg, Deutschland, Produktbeschreibung Sau3AI, Code no. 27-0913-02) partiell gespalten. Die DNA-Fragmente wurden mit shrimp alkalischer Phosphatase (Roche Molecular Biochemicals, Mannheim, Deutschland, Produktbeschreibung SAP, Code no. 1758250) dephosphoryliert. Die DNA des Cosmid-Vektors SuperCos1 (Wahl et al. (1987) Proceedings of the National Academy of Sciences USA 84:2160-2164), bezogen von der Firma Stratagene (La Jolla, USA, Produktbeschreibung SuperCosl Cosmid Vektor Kit, Code no. 251301) wurde mit dem Restriktionsenzym XbaI (Amersham Pharmacia, Freiburg, Deutschland, Produktbeschreibung XbaI, Code no. 27-0948-02) gespalten und ebenfalls mit shrimp alkalischer Phosphatase dephosphoryliert. Anschließend wurde die Cosmid-DNA mit dem Restriktionsenzym BamHI (Amersham Pharmacia, Freiburg, Deutschland, Produktbeschreibung BamHI, Code no. 27-0868-04) gespalten. Die auf diese Weise behandelte Cosmid-DNA wurde mit der behandelten ATCC 13032-DNA gemischt und der Ansatz mit T4-DNA-Ligase (Amersham Pharmacia, Freiburg, Deutschland, Produktbeschreibung T4-DNA-Ligase, Code no.27-0870-04) behandelt. Das Ligationsgemisch wurde anschließend mit Hilfe des Gigapack II XL Packing Extracts (Stratagene, La Jolla, USA, Produktbeschreibung Gigapack II XL Packing Extract, Code no. 200217) in Phagen verpackt. Zur Infektion des E. coli Stammes NM554 (Raleigh et al. 1988, Nucleic Acid Research 16:1563-1575) wurden die Zellen in 10 mM MgSO₄ aufgenommen und mit einem Aliquot der Phagensuspension vermischt. Infektion und Titerung der Cosmidbank wurden wie bei Sambrook et al. (1989, Molecular Cloning: A Laboratory Manual, Cold Spring Harbor) beschrieben durchgeführt, wobei die Zellen auf LB-Agar (Lennox, 1955, Virology, 1:190) mit 100 µg/ml Ampicillin ausplattiert wurden. Nach Inkubation über Nacht bei 37°C wurden rekombinante Einzelklone selektioniert.

### Beispiel 2

### Isolierung und Sequenzierung des ptsH-Gens

Die Cosmid-DNA einer Einzelkolonie wurde mit dem Qiaprep Spin Miniprep Kit (Product No. 27106, Qiagen, Hilden, Germany) nach Herstellerangaben isoliert und mit dem Restriktionsenzym Sau3AI (Amersham Pharmacia, Freiburg, Deutschland, Produktbeschreibung Sau3AI, Product No. 27-0913-02) partiell gespalten. Die DNA-Fragmente wurden mit shrimp alkalischer Phosphatase (Roche Molecular Biochemicals, Mannheim, Deutschland, Produktbeschreibung SAP, Product No. 1758250) dephosphoryliert. Nach gelelektrophoretischer Auftrennung erfolgte die Isolierung der Cosmidfragmente im Größenbereich von 1500 bis 2000 bp mit dem QiaExII Gel Extraction Kit (Product No. 20021, Qiagen, Hilden, Germany). Die DNA des Sequenziervektors pZero-1 bezogen von der Firma Invitrogen (Groningen, Niederlande, Produktbeschreibung Zero Background Cloning Kit, Product No. K2500-01) wurde mit dem Restriktionsenzym BamHI (Amersham Pharmacia, Freiburg, Deutschland, Produktbeschreibung BamHI, Product No. 27-0868-04) gespalten. Die Ligation der Cosmidfragmente in den Sequenziervektor pZero-1 wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5αMCR (Grant, 1990, Proceedings of the National Academy of Sciences U.S.A., 87:4645-4649) elektroporiert (Tauch et al. 1994, FEMS Microbiol Letters, 123:343-7) und auf LB-Agar (Lennox, 1955, Virology, 1:190) mit 50 µg/ml Zeocin ausplattiert. Die Plasmidpräparation der rekombinanten Klone erfolgte mit dem Biorobot 9600 (Product No. 900200, Qiagen, Hilden, Deutschland). Die Sequenzierung erfolgte nach der Dideoxy-Kettenabbruch-Methode von Sanger et al. (1977, Proceedings of the National Academy of Sciences U.S.A., 74:5463-5467) mit Modifikationen nach Zimmermann et al. (1990, Nucleic Acids Research, 18:1067). Es wurde der "RR dRhodamin Terminator Cycle Sequencing Kit" von PE Applied Biosystems (Product No. 403044, Weiterstadt, Deutschland) verwendet. Die gelelektrophoretische Auftrennung und Analyse der Sequenzierreaktion erfolgte in einem "Rotiphorese NF Acrylamid/Bisacrylamid" Gel (29:1) (Product No. A124.1, Roth, Karlsruhe, Germany) mit dem "ABI Prism 377" Sequenziergerät von PE Applied Biosystems (Weiterstadt, Deutschland).

Die erhaltenen Roh-Sequenzdaten wurden anschließend unter Anwendung des Staden-Programpakets (1986, Nucleic Acids Research, 14:217-231) Version 97-0 prozessiert. Die Einzelsequenzen der pZerol-Derivate wurden zu einem zusammenhängenden Contig assembliert. Die computergestützte Kodierbereichsanalyse wurde mit dem Programm XNIP (Staden, 1986, Nucleic Acids Research, 14:217-231) angefertigt. Weitere Analysen wurden mit den "BLAST search programs" (Altschul et al., 1997, Nucleic Acids Research, 25:3389-3402), gegen die non-redundant Datenbank des "National Center for Biotechnology Information" (NCBI, Bethesda, MD, USA) durchgeführt.

Die erhaltene Nukleotidsequenz ist in SEQ ID NO 1 dargestellt. Die Analyse der Nukleotidsequenz ergab ein offenes Leseraster von 267 Basenpaaren, welches als ptsH-Gen bezeichnet wurde. Das ptsH-Gen codiert für ein Protein von 89 Aminosäuren.

### Beispiel 3

### Herstellung eines Shuttlevektors pEC-K18mob2ptsHexp zur Verstärkung des ptsH-Gens in C. glutamicum

### 3.1. Klonierung des ptsH Gens in den Vektor pCR®Blunt II

Aus dem Stamm ATCC 13032 wurde nach der Methode von Eikmanns et al. (Microbiology 140: 1817-1828 (1994)) chromosomale DNA isoliert. Aufgrund der aus Beispiel 2 für C. glutamicum bekannten Sequenz des ptsH-Gens wurden die folgenden Oligonukleotide für die Polymerase Kettenreaktion ausgewählt:
ptsHexp1: 5` ACC ACT GGT GCA ATC TCC AT 3´ ptsHexp2:

5` TTT ACT CAG CGT CAA GGT CC 3´

Die dargestellten Primer wurden von der Firma ARK Scientific GmbH Biosystems (Darmstadt, Deutschland) synthetisiert und nach der Standard-PCR-Methode von Innis et al. (PCR protocols. A Guide to Methods and Applications, 1990, Academic Press) mit Pwo-Polymerase der Firma Roche Diagnostics GmbH (Mannheim, Deutschland) die PCR Reaktion durchgeführt. Mit Hilfe der Polymerase-Kettenreaktion ermöglichen die Primer die Amplifikation eines 686 bp großen DNA-Fragmentes, welches das ptsH-Gen mit der potentiellen Promotor-Region trägt. Die DNA Sequenz des amplifizierten DNA Fragmentes wurde durch Sequenzierung überprüft.

Das amplifizierte DNA Fragment wurde mit dem Zero Blunt™ Kit der Firma Invitrogen Corporation(Carlsbad, CA, USA; Katalog Nummer K2700-20) in den Vektor pCR®Blunt II (Bernard et al., Journal of Molecular Biology, 234: 534-541 (1993)) ligiert.

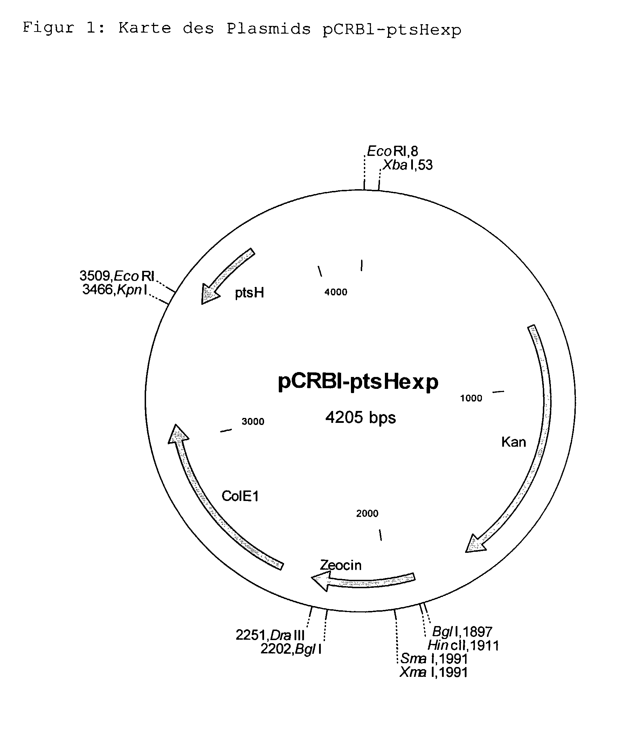

Anschließend wurde der E. coli Stamm TOP10 mit dem Ligationsansatz (Hanahan, In: DNA Cloning. A Practical Approach. Vol.I., IRL-Press, Oxford, Washington DC, USA, 1985) elektroporiert. Die Selektion von Plasmid-tragenden Zellen erfolgte durch Ausplattieren des Transformationsansatzes auf LB Agar (Sambrook et al., Molecular Cloning: A Laboratory Manual. 2^{nd} Ed., Cold Spring Harbor Laboratory Press, Cold Spring Harbor, N.Y., 1989), der mit 25 mg/l Kanamycin supplementiert worden war. Plasmid-DNA wurde aus einer Transformante mit Hilfe des QIAprep Spin Miniprep Kit der Firma Qiagen isoliert und durch Restriktion mit dem Restriktionsenzym EcoRI und anschließender Agarosegel-Elektrophorese (0,8%) überprüft. Das Plasmid wurde pCRBl-ptsHexp genannt und ist in Figur 1 dargestellt.

### 3.2. Herstellung des E. coli - C. glutamicum Shuttle Vektors pEC-K18mob2

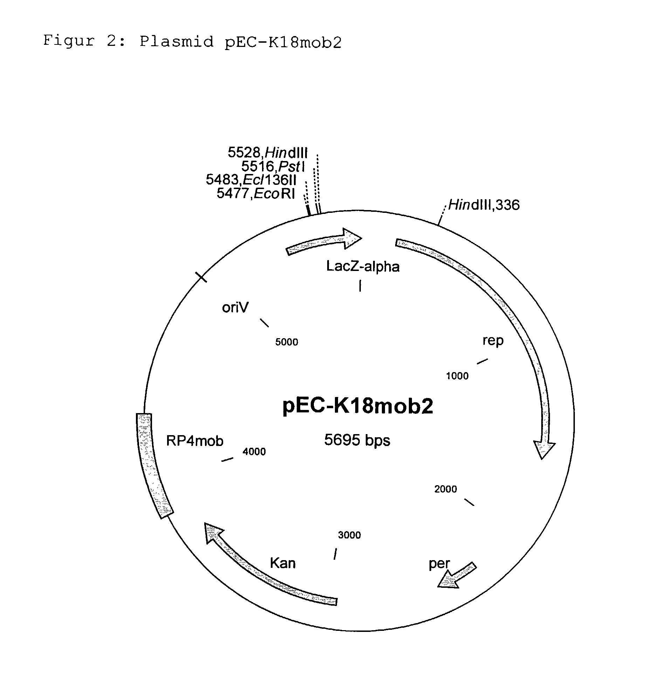

Nach dem Stand der Technik wurde der E. coli - C. glutamicum Shuttle-Vektor konstruiert. Der Vektor enthält die Replikationsregion rep des Plasmids pGAl einschließlich des Replikationseffectors per (US-A- 5,175,108; Nesvera et al., Journal of Bacteriology 179, 1525-1532 (1997)), das Kanamycinresistenz vermittelnde aph(3')-IIa-Gen des Transposons Tn5 (Beck et al., Gene 19, 327-336 (1982)), die Replikationsregion oriV des Plasmids pMB1 (Sutcliffe, Cold Spring Harbor Symposium on Quantitative Biology 43, 77-90 (1979)), das lacZα Genfragment einschließlich des lac-Promotors und einer Mehrfachklonierschnittstelle ("multiple cloning site", mcs) (Norrander, J.M. et al., Gene 26, 101-106 (1983)) und die mob-Region des Plasmids RP4 (Simon et al., Bio/Technology 1:784-791 (1983)). Der konstruierte Vektor wurde in den E. coli Stamm DH5αmcr (Hanahan, In: DNA Cloning. A Practical Approach. Vol. I, IRL-Press, Oxford, Washington DC, USA) transformiert. Die Selektion von Plasmid-tragenden Zellen erfolgte durch Ausplattieren des Transformationsansatzes auf LB Agar (Sambrook et al., Molecular Cloning: A Laboratory Manual. 2^{nd} Ed., Cold Spring Harbor Laboratory Press, Cold Spring Harbor, N.Y.), der mit 25 mg/l Kanamycin supplementiert worden war. Plasmid-DNA wurde aus einer Transformante mit Hilfe des QIAprep Spin Miniprep Kit der Firma Qiagen isoliert und durch Restriktion mit dem Restriktionsenzym EcoRI und HindIII mit anschließender Agarosegel-Elektrophorese (0,8%) überprüft. Das Plasmid wurde pEC-K18mob2 genannt und ist in Figur 2 dargestellt.

Folgender Mikroorganismus wurde bei der Deutschen Sammlung für Mikrorganismen und Zellkulturen (DSMZ, Braunschweig, Deutschland) gemäß Budapester Vertrag hinterlegt:
- C. glutamicum Stamm DSM 5715/pEC-K18mob2 als DSM 13245 3.3. Klonierung von ptsH in den E. coli-C. glutamicum Shuttle Vektor pEC-K18mob2

Für die Klonierung des ptsH-Gens in den in Beispiel 3.2 beschriebenen E. coli - C. glutamicum Shuttle-Vektor pEC-K18mob2 wurde Plasmid-DNA von pEC-K18mob2 mit den Restriktionsendonukleasen KpnI und XbaI vollständig geschnitten und mit alkalischer Phosphatase (Alkaline Phosphatase, Roche Diagnostics GmbH, Mannheim, Deutschland) behandelt.

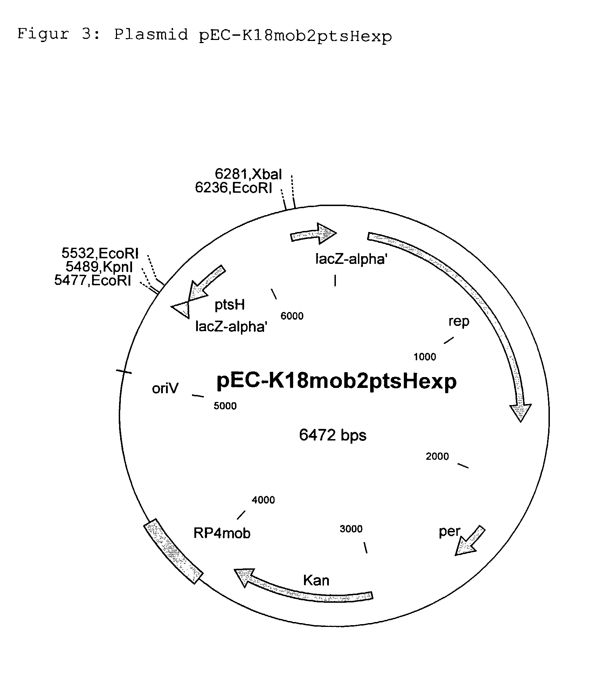

Der Vektor pCRBl-ptsHexp wurde aus Escherichia coli ToplO isoliert und mit den Restriktionsendonukleasen KpnI und XbaI vollständig geschnitten und das 788 bp große Fragment mit dem ptsH-Gen aus einem 0,8%-igen Agarosegel aufgereinigt (QIAquick Gel Extraction Kit der Firma Qiagen, Hilden, Deutschland). Anschließend wurde das Fragment mit dem ptsH-Gen mit dem Vektor pEC-K18mob2 ligiert (T4-Ligase, Roche Diagnostics GmbH, Mannheim; Deutschland). Der Ligationsansatz wurde in den E. coli Stamm DH5αmcr (Hanahan, In: DNA Cloning. A Practical Approach. Vol.I. IRL-Press, Oxford, Washington DC, USA) transformiert. Die Selektion von Plasmid-tragenden Zellen erfolgte durch Ausplattieren des Transformationsansatzes auf LB Agar (Sambrook et al., Molecular Cloning: A Laboratory Manual. 2^{nd} Ed. Cold Spring Harbor Laboratory Press, Cold Spring Harbor, N.Y., 1989), der mit 25 mg/l Kanamycin supplementiert worden war. Plasmid-DNA wurde aus einer Transformante mit Hilfe des QIAprep Spin Miniprep Kit der Firma Qiagen (Hilden, Deutschland) isoliert und durch Behandlung mit dem Restriktionsenzym EcoRI mit anschließender Agarosegel-Elektrophorese überprüft. Das Plasmid wurde pEC-K18mob2ptsHexp genannt und ist in Abbildung 3 dargestellt.

Der Stamm wurde als E. coli DH5amcr/pEC-Kl8mob2ptsHexp bezeichnet und in Form einer Reinkultur am 28. November 2000 bei der Deutschen Sammlung für Mikrorganismen und Zellkulturen (DSMZ, Braunschweig, Deutschland) als DSM 13878 gemäß Budapester Vertrag hinterlegt.

### Beispiel 4

### Transformation des Stammes DSM5715 mit dem Plasmid pEC-K18mob2ptsHexp

Der Stamm DSM5715 wurde mit dem Plasmid pEC-K18mob2ptsHexp unter Anwendung der von Liebl et al., (FEMS Microbiology Letters, 53:299-303 (1989)) beschriebenen Elektroporationsmethode transformiert. Die Selektion der Transformanten erfolgte auf LBHIS Agar bestehend aus 18,5 g/l Brain-Heart Infusion Boullion, 0,5 M Sorbitol, 5 g/l Bacto-Trypton, 2,5 g/l Bacto-Yeast-Extract, 5 g/l NaCl und 18 g/l Bacto-Agar, der mit 25 mg/l Kanamycin supplementiert worden war. Die Inkubation erfolgte für 2 Tage bei 33°C.

Plasmid-DNA wurde aus einer Transformante nach den üblichen Methoden isoliert (Peters-Wendisch et al., 1998, Microbiology, 144, 915 - 927), mit der Restriktionsendonuklease EcoRI geschnitten und das Plasmid durch anschließende Agarosegel-Elektrophorese überprüft. Der erhaltene Stamm wurde DSM5715/pEC-Kl8mob2ptsHexp genannt.

### Beispiel 5

### Herstellung von Lysin

Der in Beispiel 4 erhaltene C. glutamicum Stamm
DSM5715/pEC-K18mob2ptsHexp wurde in einem zur Produktion von Lysin geeigneten Nährmedium kultiviert und der Lysingehalt im Kulturüberstand bestimmt.

Dazu wurde der Stamm zunächst auf Agarplatte mit dem entsprechenden Antibiotikum (Hirn-Herz-Agar mit Kanamycin (25 mg/l)) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur wurde das Vollmedium Cg III verwendet.

| Medium Cg III | |
|---|---|
| NaCl | 2,5 g/l |
| Bacto-Pepton | 10 g/l |
| Bacto-Yeast-Extrakt | 10 g/l |
| Glucose (getrennt autoklaviert) | 2% (w/v) |
| Der pH-Wert wurde auf pH 7.4 eingestellt | |

Diesem wurde Kanamycin (25 mg/1) zugesetzt. Die Vorkultur wurde 16 Stunden bei 33°C bei 240 rpm auf dem Schüttler inkubiert. Von dieser Vorkultur wurde eine Hauptkultur angeimpft, so daß die Anfangs-OD (660 nm) der Hauptkultur 0,05 betrug. Für die Hauptkultur wurde das Medium MM verwendet.

| Medium MM | |
|---|---|
| CSL (Corn Steep Liquor) | 5 g/l |
| MOPS (Morpholinopropansulfonsäure) | 20 g/l |
| Glucose (getrennt autoklaviert) | 100 g/l |
| (NH₄)₂SO₄ | 25 g/l |
| KH₂PO₄ | 0,1 g/l |
| MgSO₄ ^{*} 7 H₂O | 1,0 g/l |
| CaCl₂ ^{*} 2 H₂O | 10 mg/l |
| FeSO₄ ^{*} 7 H₂O | 10 mg/l |
| MnSO₄ ^{*} H₂O | 5,0mg/l |
| Biotin (sterilfiltriert) | 0,3 mg/l |
| Thiamin ^{*} HCl (sterilfiltriert) | 0,2 mg/l |
| L-Leucin (sterilfiltriert) | 0,1 g/l |
| CaCO₃ | 25 g/l |

CSL, MOPS und die Salzlösung wurden mit Ammoniakwasser auf pH 7 eingestellt und autoklaviert. Anschließend wurden die sterilen Substrat- und Vitaminlösungen zugesetzt, sowie das trocken autoklavierte CaCO₃.

Die Kultivierung erfolgt in 10 ml Volumen in einem 100 ml Erlenmeyerkolben mit Schikanen. Es wurde Kanamycin (25 mg/l) zugesetzt. Die Kultivierung erfolgte bei 33°C und 80% Luftfeuchte.

Nach 48 Stunden und 72 Stunden wurde die OD bei einer Meßwellenlänge von 660 nm mit dem Biomek 1000 (Beckmann Instruments GmbH, München) ermittelt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt.

In Tabelle 1 ist das Ergebnis des Versuchs dargestellt.

**Tabelle 1**

| Stamm | OD (660 nm) | Lysin-HCl g/l |
|---|---|---|
| DSM5715/pEC-K18mob2 (48 Stunden) | 11,4 | 14,14 |
| DSM5715/pEC-Kl8mob2ptsHexp (48 Stunden) | 10,7 | 15,98 |
| DSM5715/pEC-K18mob2mob2 (72 Stunden) | 10,1 | 15,24 |
| DSM5715/pEC-Kl8mob2ptsHexp (72 Stunden) | 10,0 | 17,13 |

Folgende Figuren sind beigefügt:
- Figur 1:: Karte des Plasmids pCRBl-ptsHexp
- Figur 2:: Karte des Plasmids pEC-K18mob2
- Figur 3:: Karte des Plasmids pEC-K18mob2ptsHexp

Die verwendeten Abkürzungen und Bezeichnungen haben folgende Bedeutung:
- Kan:: Resistenzgen für Kanamycin
- Zeocin:: Zeocin Resistenz-Gen
- ptsH:: ptsH-Gen aus C. glutamicum
- ColE1:: Replikationsursprung des Plasmides ColE1
- lacZ-alpha:: lacZ-Genfragment aus E.coli
- lacZ-alpha':: Fragment des lacZ-Genfragmentes aus E.coli
- per:: Gen zur Kontrolle der Kopienzahl aus pGA1
- oriV:: ColEl-ähnlicher Origin aus pMB1
- rep:: Plasmidkodierte Replikationsregion aus C. glutamicum Plasmid pGA1
- RP4mob:: RP4-Mobilisierungs-Site

- EcoRI:: Schnittstelle des Restriktionsenzyms EcoRI
- HindIII:: Schnittstelle des Restriktionsenzyms HindIII
- KpnI:: Schnittstelle des Restriktionsenzyms KpnI
- XbaI:: Schnittstelle des Restriktionsenzyms XbaI

## Patentansprüche

**1.** Isoliertes Polynukleotid aus coryneformen Bakterien, enthaltend eine Polynukleotidsequenz, ausgewählt aus der Gruppe
a) Polynukleotid, das mindestens zu 70 % identisch ist mit einem Polynukleotid, das für ein Polypeptid codiert, das die Aminosäuresequenz von SEQ ID No. 2 enthält,
b) Polynukleotid, das für ein Polypeptid codiert, das eine Aminosäuresequenz enthält, die zu mindestens 70 % identisch ist mit der Aminosäuresequenz von SEQ ID No.2,
c) Polynukleotid, das komplementär ist zu den Polynukleotiden von a) oder b), und
d) Polynukleotid, enthaltend mindestens 15 aufeinanderfolgende Nukleotide der Polynukleotidsequenz von a), b) oder c).

**2.** Polynukleotid gemäß Anspruch 1,
wobei das Polynukleotid eine in coryneformen Bakterien replizierbare, bevorzugt rekombinante DNA ist.

**3.** Polynukleotid gemäß Anspruch 1,
wobei das Polynukleotid eine RNA ist.

**4.** Replizierbare DNA gemäß Anspruch 2, enthaltend
(i) die Nukleotidsequenz, gezeigt in SEQ ID No. 1, oder
(ii) mindestens eine Sequenz, die der Sequenz (i) innerhalb des Bereichs der Degeneration des genetischen Codes entspricht, oder
(iii) mindestens eine Sequenz, die mit der zur Sequenz (i) oder (ii) komplementären Sequenz hybridisiert, und gegebenenfalls
(iv) funktionsneutrale Sinnmutationen in (i).

**5.** Polynukleotidsequenz gemäß Anspruch 2, das für ein Polypeptid codiert, das die in SEQ ID No. 2 dargestellte Aminosäuresequenz enthält.

**6.** Vektor, enthaltend eine Polynukleotidsequenz nach Anspruch 1.

**7.** Coryneformes Bakterium, enthaltend einen Vektor nach Anspruch 6.

**9.** Verfahren zur fermentativen Herstellung von L-Aminosäuren,
**dadurch gekennzeichnet,**
daß man folgende Schritte durchführt:
a) Fermentation von die L-Aminosäure produzierenden coryneformen Bakterien, in denen man zumindest das für die Komponente H des Phosphotransferase-Systems codierende Gen verstärkt, insbesondere überexprimiert,
b) Anreicherung der L-Aminosäure im Medium oder in den Zellen der Bakterien und
c) Isolieren der L-Aminosäure.

**10.** Verfahren gemäß Anspruch 9,
**dadurch gekennzeichnet,**
daß man Bakterien einsetzt, in denen man zusätzlich weitere Gene des Biosyntheseweges der gewünschten L-Aminosäure verstärkt.

**11.** Verfahren gemäß Anspruch 9,
**dadurch gekennzeichnet,**
daß man Bakterien einsetzt, in denen die Stoffwechselwege zumindest teilweise ausgeschaltet sind, die die Bildung der L-Aminosäure verringern.

**12.** Verfahren gemäß Anspruch 9,
**dadurch gekennzeichnet,**
daß man einen mit einem Plasmidvektor transformierten Stamm einsetzt, und der Plasmidvektor die Nukleotidsequenz des für die Komponente H des Phosphotransferase-Systems codierenden Gens trägt.

**13.** Verfahren gemäß einem oder mehreren der Ansprüche 9 bis 12,
**dadurch gekennzeichnet,**
daß man coryneforme Bakterien verwendet, die L-Lysin herstellen.

**14.** Verfahren gemäß Anspruch 10,
**dadurch gekennzeichnet**,
daß man gleichzeitig eines oder mehrere der Gene, ausgewählt aus der Gruppe
das für die Dihydrodipicolinat-Synthase kodierende dapA-Gen,
das für die Pyruvat-Carboxylase kodierende pyc-Gen,
das für die Triosephosphat-Isomerase kodierende tpi-Gen,
das für die Glyceraldehyd-3-Phosphat-Dehydrogenase kodierende gap-Gen,
das für die Komponente M des Phospoenolpyruvat-Zucker-Phosphotransferase System (ptsM) kodierende ptsM-Gen
das für die 3-Phosphoglycerat-Kinase kodierende pgk-Gen, und
das für den Lysin-Export kodierende lysE-Gen,
verstärkt, insbesondere überexprimiert oder amplifiziert.

**15.** Verfahren gemäß Anspruch 11,
**dadurch gekennzeichnet,**
daß man für die Herstellung von L-Lysin Bakterien fermentiert, in denen man gleichzeitig eines oder mehrere der Gene, ausgewählt aus der Gruppe
das für die Phosphoenolpyruvat-Carboxykinase codierende pck-Gen,
das für die Glucose-6-Phosphat-Isomerase kodierende pgi-Gen,
das für die Pyruvat-Oxidase kodierende poxB-Gen
abschwächt.

**16.** Verfahren gemäß einem oder mehreren der vorhergehenden Ansprüche,
**dadurch gekennzeichnet,**
daß man Mikroorganismen der Gattung Corynebacterium glutamicum einsetzt.

**17.** Verwendung von Polynukleotidsequenzen gemäß Anspruch 1 als Primer zur Herstellung der DNA von Genen, die für das ptsH Genprodukt codieren, durch die Polymerase-Kettenreaktion.

**18.** Verwendung von Polynukleotidsequenzen gemäß Anspruch 1 als Hybridisierungssonden.


SEQUENZPROTOKOLL

<110> Degussa-Hüls AG

<120> Neue für das ptsH Gen kodierende Nukleotidsequenzen

<130> 990219 BT

<140>
<141>

<160> 2

<170> PatentIn Ver. 2.1

<210> 1
<211> 480
<212> DNA
<213> Corynebacterium glutamicum

<220>
<221> CDS
<222> (163)..(429)

<400> 1
ggacattgtt tttgcttccg gtaacgtggc aaaacgaaca atgtctcact agactaaagt 60

gagatcgaca ttaaatcccc tcccttgggg ggtttaacta acaaatcgct gcgccctaat 120

ccgttcggat taacggcgta gcaacacgaa aggacacttt cc atg gct tcc aag 174
Met Ala Ser Lys
1

act gta acc gtc ggt tcc tcc gtt ggc ctg cac gca cgt cca gca tcc 222
Thr Val Thr Val Gly Ser Ser Val Gly Leu His Ala Arg Pro Ala Ser
5                   10                  15                  20

atc atc gct gaa gcg gct gct gag tac gac gac gaa atc ttg ctg acc 270
Ile Ile Ala Glu Ala Ala Ala Glu Tyr Asp Asp Glu Ile Leu Leu Thr
                25                  30                  35

ctg gtt ggc tcc gat gat gac gaa gag acc gac gcg tcc tct tcc ctc 318
Leu Val Gly Ser Asp Asp Asp Glu Glu Thr Asp Ala Ser Ser Ser Leu
            40                  45                  50

atg atc atg gcg ctg ggc gca gag cac ggc aac gaa gtt acc gtc acc 366
Met Ile Met Ala Leu Gly Ala Glu His Gly Asn Glu Val Thr Val Thr
        55                  60                  65

tcc gac aac gct gaa gct gtt gag aag atc gct gcg ctt atc gca cag 414
Ser Asp Asn Ala Glu Ala Val Glu Lys Ile Ala Ala Leu Ile Ala Gln
    70                  75                  80

gac ctt gac gct gag taaacaacgc tctgcttgtt aaaagctcgt tagaagcttg 469
Asp Leu Asp Ala Glu
85

ttaaaagcgg t                                                          480

<210> 2
<211> 89
<212> PRT
<213> Corynebacterium glutamicum

<400> 2
Met Ala Ser Lys Thr Val Thr Val Gly Ser Ser Val Gly Leu His Ala
1               5                   10                  15

Arg Pro Ala Ser Ile Ile Ala Glu Ala Ala Ala Glu Tyr Asp Asp Glu
            20                  25                  30

Ile Leu Leu Thr Leu Val Gly Ser Asp Asp Asp Glu Glu Thr Asp Ala
        35                  40                  45

Ser Ser Ser Leu Met Ile Met Ala Leu Gly Ala Glu His Gly Asn Glu
    50                  55                  60

Val Thr Val Thr Ser Asp Asn Ala Glu Ala Val Glu Lys Ile Ala Ala
65                  70                  75                  80

Leu Ile Ala Gln Asp Leu Asp Ala Glu
                85